# EUROPEAN PATENT SPECIFICATION

(11) **EP 1 806 366 B1**
(45) Date of publication and mention of the grant of the patent: **05.09.2012**
(21) Application number: 05800367.4
(22) Date of filing: 31.10.2005
(51) Int. Cl.: C08B 30/18, C08L 3/02

(54) **PROCESS FOR PRODUCING INDIGESTIBLE DEXTRIN CONTAINING ISOMERIZED SUGAR**
VERFAHREN ZUR HERSTELLUNG FÄULNISBESTÄNDIGEN DEXTRINS MIT ISOMERISIERTEM ZUCKER
PROCÉDÉ DE SYNTHÈSE DE DEXTRINE INDIGESTIBLE CONTENANT UN SUCRE ISOMÉRISÉ

(30) Priority: 29.10.2004 JP 2004316301; 01.07.2005 JP 2005193842
(43) Date of publication of application: 11.07.2007
(73) Proprietor: MATSUTANI CHEMICAL INDUSTRY CO., LTD., Itami City, Hyogo 664-8508 (JP)
(72) Inventor: Ohkuma, Kazuhiro, Sanda-shi, Hyogo 669-1546 (JP); Matsuda, Isao, Itami-shi, Hyogo 664-0873 (JP); Ichihara, Takashi, Kobe-shi, Hyogo 6580022 (JP); Yamada, Koji, Nishinomiya-shi Hyogo 662-0954 (JP)
(74) Representative: Hubert, Philippe
(86) International application number: PCT/JP2005/019987
(87) International publication number: WO 2006/046738

(56) References cited:
- EP-A1- 0 530 111
- WO-A1-93/05663
- JP-A- 02 154 664
- JP-A- 08 070 842
- JP-A- 11 209 403
- JP-A- 2004 248 673
- JP-A- 2005 287 454
- US-A- 4 605 619
- KISHIMOTO YUKA ET AL: "Acute toxicity and mutagenicity study on branched corn syrup and evaluation of its laxative effect in humans", JOURNAL OF NUTRITIONAL SCIENCE AND VITAMINOLOGY, vol. 47, no. 2, April 2001 (2001-04), pages 126-131, XP009149371, ISSN: 0301-4800
- YASUHIRO KIMURA ET AL: "Nondigestible oligosaccharides do not increase accumulation of lipid soluble environmental contaminants by mice.", THE JOURNAL OF NUTRITION, vol. 132, no. 1, 1 January 2002 (2002-01-01), pages 80-7, XP55000701, ISSN: 0022-3166
- KALEVI VISURI ET AL: "Enzymatic production of high fructose corn syrup (HFCS) containing 55% fructose in aqueous ethanol", BIOTECHNOLOGY AND BIOENGINEERING, vol. 30, no. 7, 1 November 1987 (1987-11-01), pages 917-920, XP55000699, ISSN: 0006-3592, DOI: 10.1002/bit.260300715

## Description

### Technical Field

The present invention relates to producing indigestible dextrin comprising isoglucose

### Background Art

Indigestible dextrin has effects of regulating intestinal function, preventing sudden increases in blood sugar after eating, and reducing neutral fats and cholesterol in blood serum with long-term intake, and it is widely used as a functional food product ingredient. However, if the number of steps in a method for preparation of indigestible dextrin is small, the content of digestion-resistant components in the product is low. On the other hand, if the number of steps in a method for preparation of indigestible dextrin is large (for example, such a step wherein α-amylase and glucoamylase are acted on pyrodextrin to convert digestible fractions into glucose, followed by separation of digestion-resistant fractions by chromatography or a membrane), an yield of digestion-resistant product is about 40% and production cost is high (Patent Documents 1 and 2). In practice, however, a search for food product applications reveals that there are very many cases in which indigestible dextrin used in beverages, confectioneries, desserts, and the like is used along with so-called isoglucose such as glucose-fructose liquid sugar syrup (a mass ratio of glucose to fructose = 58:42) or a fructose-glucose liquid sugar syrup (a mass ratio = 45:55).

When foods and beverages comprising both indigestible dextrin and isoglucose are produced, indigestible dextrin and isoglucose which are separately prepared are premixed at desired concentrations and then added to the foods, or these are added separately. However, conventional methods used to prepare foods and beverages comprising both indigestible dextrin and isoglucose are deficient because the methods need complicated steps, and high production costs.
It may be possible to obtain indigestible dextrin comprising isoglucose by acting glucose isomerase on glucose that is a byproduct in a step of producing indigestible dextrin from pyrodextrin, or by adding fructose. However, it has not been proposed any method for production of indigestible dextrin comprising isoglucose based on the above concept.
Indigestible dextrin comprising glucose can be obtained as an intermediate product in a step of producing indigestible dextrin (Patent Document 2), but typically the glucose purity is about 50 percent.
Meanwhile, in conventional method for producing isoglucose, glucose isomerase is acted on not less than 95% high-purity glucose. This is because that isomerization reaction is an equilibrium reaction, the maximum yield of fructose from glucose is 50%, and the higher the purity of the glucose in the feedstock liquid sugar, the easier the isomerization reaction progresses (Non-Patent Document 1).
Therefore, it is difficult to predict whether or not an isomerization reaction progresses sufficiently in a process for producing indigestible dextrin comprising isoglucose by acting glucose isomerase on a glucose solution comprising low-purity indigestible dextrin such as that obtained as an intermediate product in a method of the production of indigestible dextrin.

Patent Document 1:JP-A-H2-145169
Patent Document 2: JP-AH2-154664
Non-Patent Document 1: Starch Science Encyclopedia, pp. 464-466, FUWA, Hidetsugu, and 3 others (editors), Asakura Shoten, 2003.
EP 0 530 111 A1 , WO 93/05663 A1 and Yasuhiro Kimura et al.: "Nondigestable oligosaccharides do not increase accumulation of lipid soluble environmental contaminants by mice.", The Journal of Nutrition, vol. 132, no 1, 1 January 2002 (2002-01-01), pages 80 - 7, disclose the generation of indigestible dextrin, Kalevi Visuri et al.: "Enzymatic production of high fructose corn syrup (HFCS) containing 55 % fructose in aqueous ethanol", Biotechnology and Bioengineering, vol. 30, no. 7, 1 November 1987 (1987-11-01), pages 917 - 920, US 4 605 619 A and JP 2004 248 673 A show the enzymatic generation of high fructose corn syrup, and JP 11 209403 A and JP 08 070842 A the treatment of pyrodextrin with glucoamylase or alpha-amylase.

### Disclosure of the Invention

### Problems to be Solved by the Invention

An object of the present invention is to provide a low-cost method for production of indigestible dextrin comprising isoglucose that has a high value of use in foods and beverages.

### Means for Solving the Problems

The inventors have found that after converting digestible fractions in pyrodextrin comprising digestion-resistant components into glucose, glucose isomerase is acted on the thus produced indigestible dextrin comprising glucose to easily convert a portion of glucose into fructose and generate isoglucose with the same as or greater efficiency than acting glucose isomerase on a solution of glucose, to thereby efficiently produce indigestible dextrin comprising isoglucose. Based on the discovery, they arrived at the present invention. In other words, the present invention provides a method for production of indigestible dextrin comprising isoglucose at a low cost, as defined in the claims.

### Effect of the Invention

According to the present invention, the indigestible dextrin comprising isoglucose can easily be obtained by adding fructose to a mixed solution of glucose and indigestible dextrin that is obtained as an intermediate in a process of production of indigestible dextrin, or by using glucose as a substrate for glucose isomerase. Therefore, the step of separating indigestible dextrin can be omitted and it is possible to produce, at a low-cost, indigestible dextrin comprising isoglucose which is highly valuable for use in food and beverages. Moreover, it is possible to produce foods and beverages containing both indigestible dextrin and isoglucose efficiently at a low cost. Furthermore, by hydrogenating the indigestible dextrin comprising isoglucose obtained by the method of the present invention, it is possible to produce hydrogenated indigestible dextrin comprising hydrogenated isoglucose that has low energy, low sweetness, and high color stability.

### Best Modes for Carrying Out the Invention

The words "indigestible dextrin" in the present invention means a dextrin comprising digestion-resistant components in an amount of preferably 35 to 95 % by mass, more preferably 60 to 90 % by mass based on the total amount of the dextrin comprising the digestion-resistant components as measured according to the high-performance liquid chromatography method (enzyme-HPLC method) for analysis of dietary fiber specified in Ei-Shin No. 13 (Notification No. 13 by Office of Health Policy on Newly Developed Foods, MHW, regarding methods for analysis of nutritional components in nutritional labeling guidelines).
"Whiteness" of pyrodextrin used in the present invention means a relative value of a reflected light intensity of a sample to a reflected light intensity of a standard white magnesium oxide plate having a reflected light intensity value of 100 measured by a color-difference meter (available from, for example, Nippon Denshoku Industries Co., Ltd.) and the maximum value is 100.
Moreover, "isoglucose" in the present invention means a liquid sugar syrup comprising glucose and fructose. "Glucose-fructose liquid sugar syrup" in the present invention means an isoglucose in which the relative concentration of glucose is equal to or greater than that of fructose, while "fructose-glucose liquid sugar syrup" means an isoglucose in which the relative concentration of fructose is greater than that of glucose.
The method for production of indigestible dextrin comprising isoglucose in the present invention comprises step (A) wherein pyrodextrin comprising digestion-resistant components is dissolved in water, on which glucoamylase is acted to hydrolyze digestible components to glucose; then step (B) wherein fructose is added, or step (C) wherein a portion of the glucose produced is converted into fructose with glucose isomerase; or further comprises step (D) wherein the product obtained in at least one of the steps (B) and (C) is hydrogenated. The method for production of the indigestible dextrin comprising isoglucose of the present invention may comprise all the steps (A), (B), (C), and (D).

### (A) Enzymatic digestion step wherein glucoamylase is acted on pyrodextrin comprising digestion-resistant components to hydrolyze digestible components to glucose

Conventional methods can be used for production of pyrodextrin used in the present invention. However, it is preferable to use production methods that hardly generate irritating odors or unpleasant taste. Raw starches include those widely used such as corn, wheat, cassava, potato, and other commercially available processed starches.
About 1 % by mass aqueous solution of mineral acid such as sulfuric acid, nitric acid, hydrochloric acid, preferably hydrochloric acid, is added to, preferably sprayed onto raw starch in an amount of several percents by mass based on the starch, and mixed to obtain a uniform mixture, which mixture is then pre-dried at about 100 to 120 °C so that the moisture content is reduced to preferably 2 to 6 % by mass, more preferably about 3 % by mass.
Next, the mixture is roasted at a temperature of preferably 130 to 180 °C for about 0.5 to 5 hours to obtain pyrodextrin. The pyrodextrin thus obtained has preferably a DE of 1 to 10, whiteness of 55 to 65, and a content of digestion-resistant components of 50 to 65 % by mass.

Next, glucoamylase (a digestive enzyme) is acted. In order to prepare indigestible dextrin having a high content of digestion-resistant components, it is desirable to act α-amylase prior to acting the glucoamylase in the enzyme digesting step.
An example of the enzyme digesting step of the present invention will be explained. First, pyrodextrin is dissolved in water to obtain a solution of preferably 30 to 50 % by mass. A pH of the solution is adjusted with for example, sodium hydroxide aqueous solution to preferably 5.5 to 6.5, more preferably 5.8. Then, commercially-available α-amylase preparation derived from mold or bacilli is added in an amount of preferably 0.5 to 0.2 % by mass to the solution. The mixture is kept at a temperature of preferably 50 to 100 °C for preferably 0.5 to 2.0 hours. Subsequently, the mixture is cooled to preferably 40 to 60 °C and adjusted to a pH of preferably 4.5 to 5.5. Then, commercially available glucoamylase preparation is added in an amount of preferably 0.1 to 0.5 % by mass at a temperature of preferably 45 to 55 °C. The mixture is held for preferably 1 to 10 hours to convert the digestible components into glucose.
If it is not necessary to prepare indigestible dextrin having a so high content of digestion-resistant components, α-amylase may not be used in this enzyme-digesting step.
This aqueous solution may be decolorized if necessary by activated charcoal, diatomaceous earth, or the like, and moreover desalinated by ion-exchange resin or the like, and concentrated to produce an aqueous solution of indigestible dextrin comprising glucose.

### (B) Step of adding fructose and (C) step of converting a portion of the glucose produced into fructose by means of glucose isomerase

The present invention further comprises a step wherein fructose is added to the aqueous solution of indigestible dextrin comprising glucose (Step (B)), and/or a step wherein a portion of the glucose produced is converted into fructose by means of glucose isomerase (Step (C)).
In step (C), for example, an aqueous solution of indigestible dextrin comprising glucose is passed through a column packed with glucose isomerase immobilized to a carrier to continuously convert a portion of the glucose to fructose and to obtain an aqueous solution of indigestible dextrin comprising glucose-fructose liquid sugar syrup or fructose-glucose liquid sugar syrup.
This solution can be decolorized if necessary by activated charcoal, diatomaceous earth, or the like, desalinated by ion-exchange resin or the like and concentrated to obtain a highly-concentrated product. A ratio of glucose to fructose can be adjusted as appropriate by varying an amount of fructose added and the processing parameters in step (C).

### (D) Step of hydrogenating the product obtained in at least one of the steps (B) and (C).

The indigestible dextrin comprising isoglucose obtained in step (B) and/or (C) can be reduced with hydrogen to form hydrogenated indigestible dextrin comprising hydrogenated isoglucose. For example, an aqueous solution of indigestible dextrin comprising glucose-fructose liquid sugar syrup or fructose-glucose liquid sugar syrup is decolorized if necessary by activated charcoal, diatomaceous earth, or the like, desalinated by ion-exchange resin or the like, to produce a concentrated solution to which hydrogen is added using conventional methods of introducing gaseous hydrogen under pressure in the presence of Raney nickel or the like as a catalyst. After the reaction has been completed, the catalyst is removed, the product is decolorized if necessary by activated charcoal, diatomaceous earth, or the like, desalinated by ion-exchange resin or the like, and concentrated to obtain a concentrated solution of hydrogenated indigestible dextrin comprising isoglucose.
The indigestible dextrin comprising isoglucose or the hydrogenated indigestible dextrin comprising hydrogenated isoglucose thus obtained can be suitably used in the production of, for example, Japanese and Western-style cakes comprising both indigestible dextrin and isoglucose, such as carbonated beverages, fruit juice drinks, yogurt, jams, jellies, and cakes. These foods and beverages may contain the indigestible dextrin comprising isoglucose or the hydrogenated indigestible dextrin comprising hydrogenated isoglucose of the present invention in an optional amount, typically 5 to 15 % by mass.
The present invention will be explained with the following examples to which the present invention is not limited. In the following examples and comparative examples, "parts" means "parts by mass" unless otherwise indicated.

### Example 1

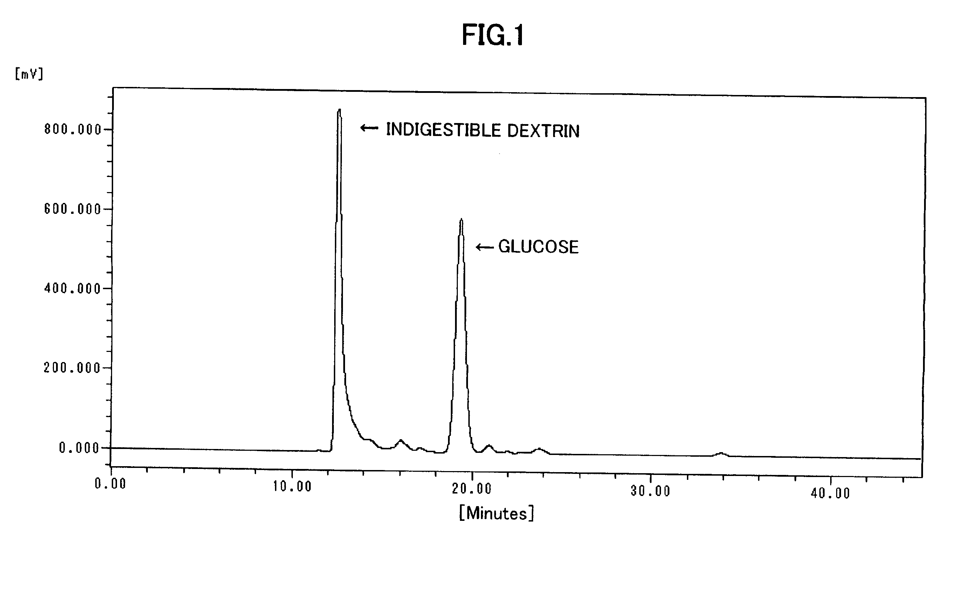

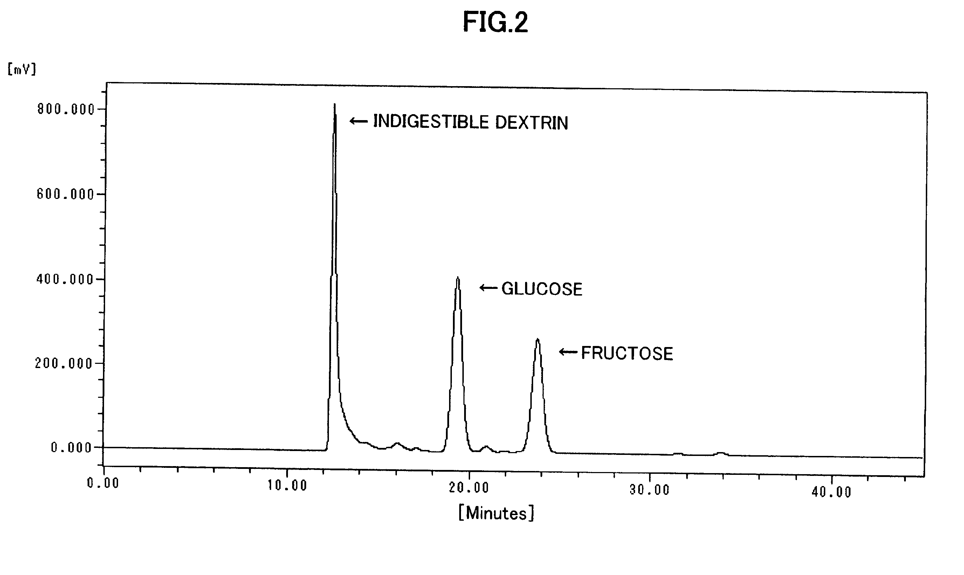

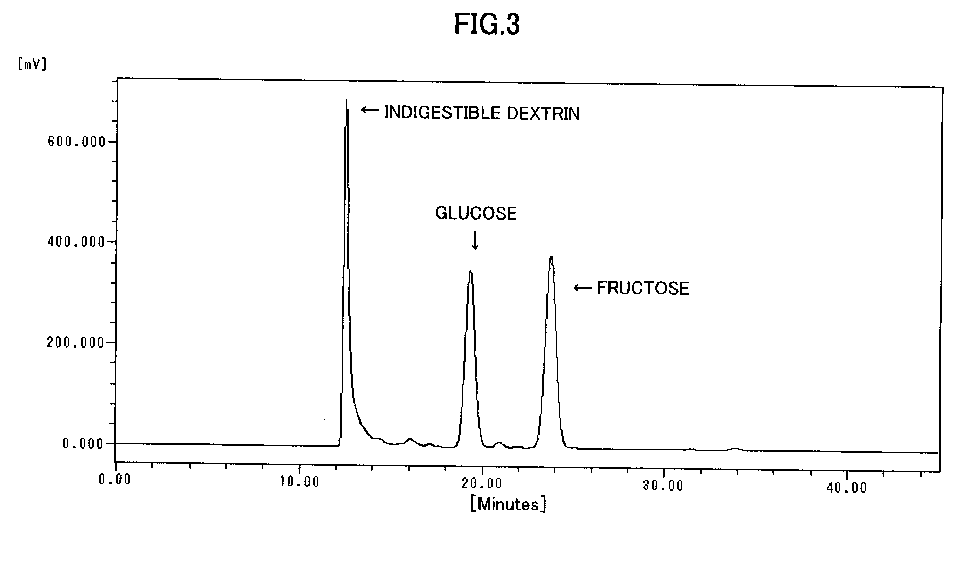

Commercially-available corn starch (1 kg) was placed in a heating device equipped with a mixer, was stirred while 30 ml of 1% hydrochloric acid was sprayed, and then mixed uniformly while gradually raising the temperature to 110 °C to obtain a preliminary dried mixture with a 3 % by mass moisture content, which was then heated for 40 minutes at 150 °C to obtain pyrodextrin with a whiteness of 60.
Water (1,000 ml) was added to 500 g of the pyrodextrin to dissolve it. To the solution, 4% sodium hydroxide solution was added to adjust a pH to 5.8, and 0.2 % by mass of α-amylase (Termamyl 60L, Novozyme Corp.) was added to hydrolyze the pyrodextrin at 95 °C for 1 hour.
After adjusting the pH to 5.0, 0.1 % by mass glucoamylase (Gluczyme NL 4.2, Amano Pharmaceutical Co., Ltd.) was added to hydrolyze the mixture at 50 °C for three hours.
To the mixture, 0.2 % by mass of activated charcoal was added and held for 30 minutes. The mixture was then passed through a diatomaceous earth filter to decolorize it, and then desalinated by ion-exchange resin and concentrated to a Brix concentration of 60% to obtain 750 g of mixed solution containing glucose and indigestible dextrin whose solid content was about 450 g. This mixed solution contained 41.4 parts of glucose and 58.6 parts of indigestible dextrin (Figure 1).
Fructose was added to this mixed solution to obtain a mixed solution of indigestible dextrin (58.6 parts), glucose (41.4 parts), and fructose (30.0 parts) (Figure 2), and a mixed solution of indigestible dextrin (58.6 parts), glucose (41.4 parts), and fructose (50.6 parts) (Figure 3).
For the analysis of indigestible dextrin, glucose, and fructose, a column for high-performance liquid chromatography (HPLC) (MCI GEL CK08EC available from Mitsubishi Chemical Co., Ltd.) was used, wherein elution was conducted at a column temperature of 80 °C with ion exchange water, and detected with an RI detector.

### Example 2

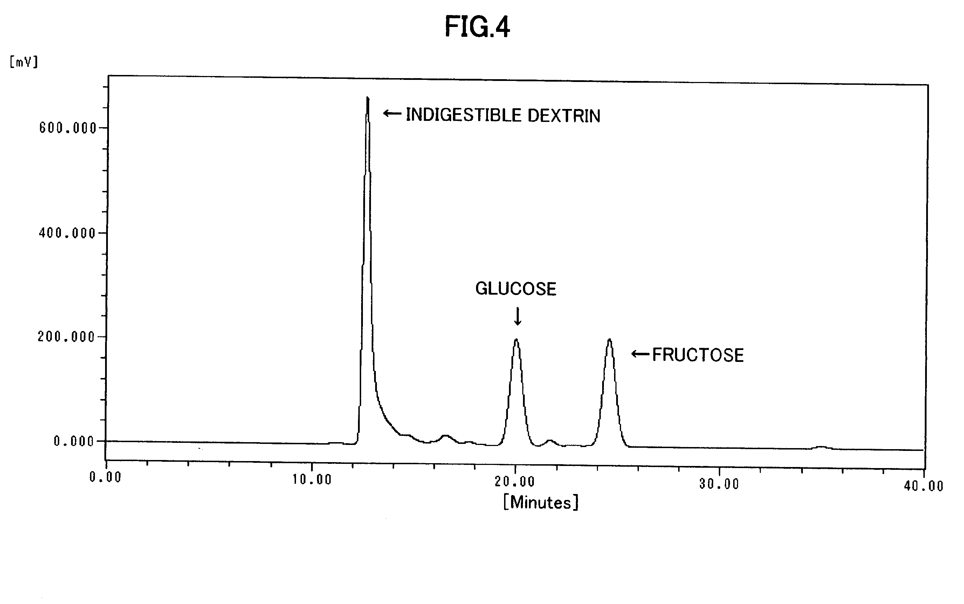

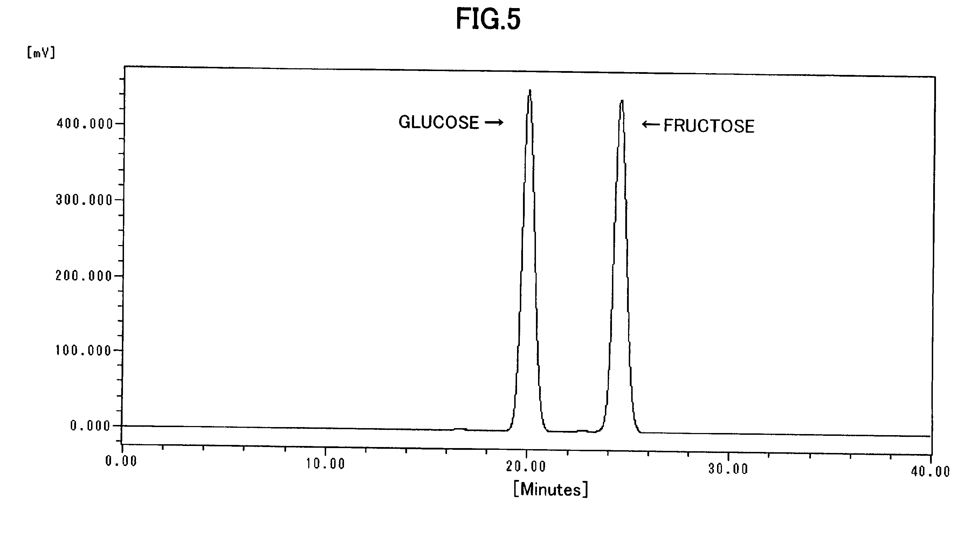

The mixed solution of glucose and indigestible dextrin obtained in Example 1 was adjusted to a Brix concentration of 10%, and was passed through a column (57 ml) packed with immobilized glucose isomerase preparation (Sweetzyme IT, Novozyme Corp.) at an SV (ml amount of liquid passed through/hr/ml column volume) of 3.5 and at a column temperature of 70 °C to obtain a mixed solution of glucose, fructose, and indigestible dextrin. This was passed through mixed ion-exchange resin (a mixture of equal amount of strongly basic ion-exchange resin and strongly acidic ion-exchange resin) and desalinated to obtain a concentrated liquid.
As shown in Figure 4, the composition of this solution was 58.6 parts of indigestible dextrin, 21.1 parts of glucose, and 20.3 parts of fructose. The isomerization rate of fructose from glucose was 49.0%.
As a control, the same experiments were performed using a glucose solution having a Brix concentration of 10%. A composition comprising 50.5 parts of glucose and 49.5 parts of fructose with an isomerization rate of 49.5% was obtained (Figure 5).
These results indicate that an isomerization reaction having the same efficiency as a single solution of glucose proceeds even if it is a glucose solution comprising indigestible dextrin.

### Example 3

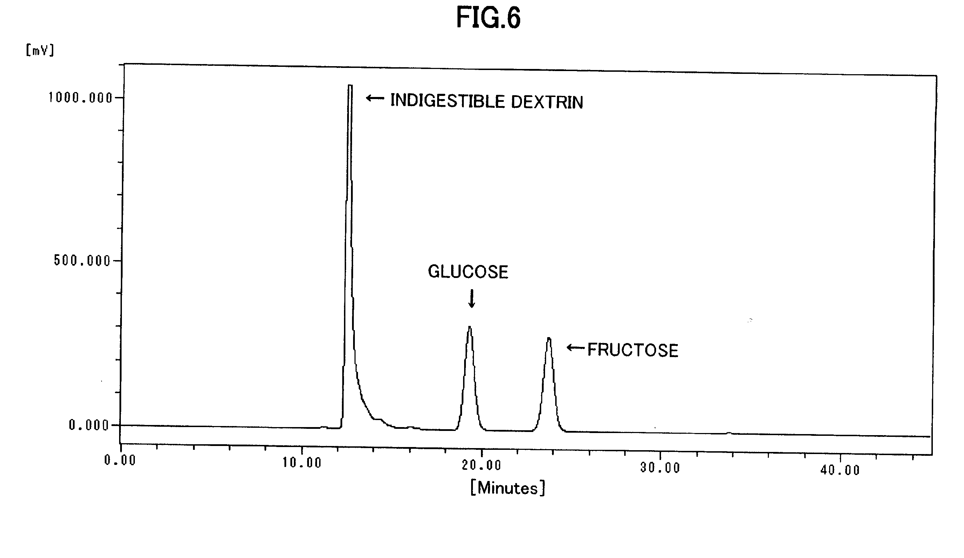

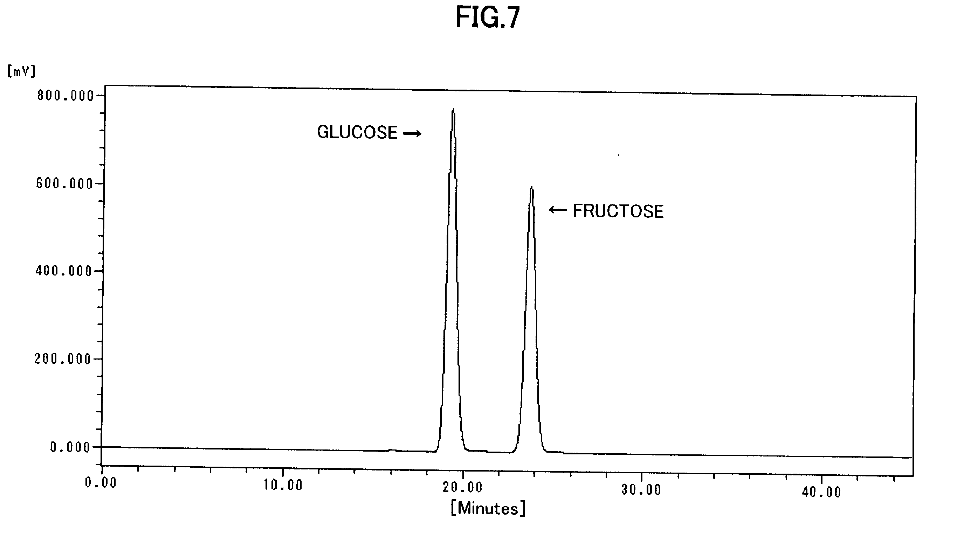

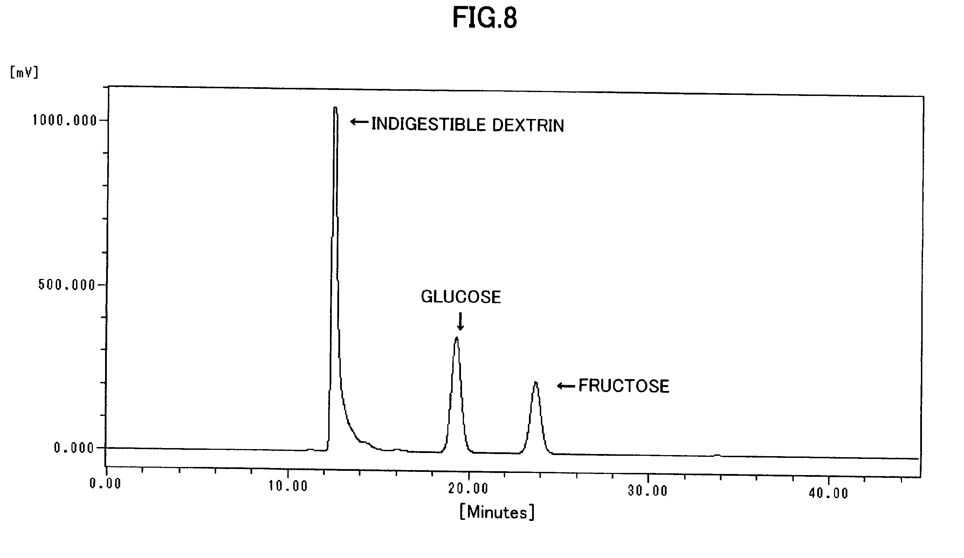

The mixed solution of glucose and indigestible dextrin obtained in Example 1 was adjusted to a Brix concentration of 45%, and was passed through a column (2000 ml) packed with immobilized glucose isomerase preparation (Sweetzyme IT, Novozyme Corp.) at an SV (ml amount of liquid passed through/hr/ml column volume) of 3.5 and at a column temperature of 55 °C to obtain a mixed solution of glucose, fructose, and indigestible dextrin. Then the mixed solution was passed through the mixed ion-exchange resin and desalinated to obtain a concentrated liquid.
As shown in Figure 6, the composition of this solution was 58.6 parts of indigestible dextrin, 21.1 parts of glucose, and 20.3 parts of fructose, and the isomerization rate of fructose from glucose was 49.0%.
As a control, the same experiments were performed for a glucose solution having a Brix concentration of 45% at an SV of 1.2. A composition containing 55 parts of glucose and 45 parts of fructose was obtained with an isomerization rate of 45.0% (Figure 7).
Moreover, the mixed solution of glucose and indigestible dextrin obtained in Example 1 was adjusted to a Brix concentration of 45%, and a similar experiment was performed at an SV of 1.75. A composition containing 58.6 parts of indigestible dextrin, 24.8 parts of glucose, and 16.6 parts of fructose was obtained with an isomerization rate of 40.1% (Figure 8).
These results indicate that by adjusting an SV, the isomerization reaction of a glucose solution comprising indigestible dextrin can be performed with greater efficiency than one using a single solution of glucose. Further, the isomerization rate (49%) of this example is comparable to the maximum isomerization rate (50%) in conventional commercial isoglucose production (Non-patent Document 1).

### Example 4

Fructose (13.7 parts) was added to 100 parts of indigestible dextrin comprising isoglucose obtained in Example 4 under SV 1.75 conditions to obtain an indigestible dextrin liquid sugar syrup comprising isoglucose with a ratio of fructose to glucose of 55:45. Similarly, 206.6 parts fructose was added to obtain an indigestible dextrin solution comprising isoglucose with a ratio of fructose to glucose of 90:10.

### Example 5

The indigestible dextrin comprising isoglucose obtained in Example 1 (indigestible dextrin: 58.6 parts, glucose: 41.4 parts, fructose: 50.6 parts) was adjusted to a Brix concentration of 50%, and a portion of it was used to prepare orangeade according to the recipe indicated in Table 1 below. The ingredients were blended, heated and filled in a container to obtain the desired beverage. This orangeade comprised 3.4 g of glucose, 4.2 g of fructose, and 4.9 g of indigestible dextrin derived from the preparation of the present invention.

**Table 1**

| Ingredient | Ratio (grams) |
|---|---|
| Indigestible dextrin comprising isoglucose | 25 |
| 5x concentrated orange juice | 5.75 |
| 5× concentrated lemon juice | 0.25 |
| Citric acid | 0.01 |
| Vitamin C | 0.01 |
| Flavor | 0.15 |
| Water | To make up to a total volume of 100 ml |

### Example 6

The solution obtained in Example 2 comprising 20.3 parts of fructose, 21.1 parts of glucose, and 58.6 parts of indigestible dextrin was adjusted to a Brix concentration of 65%. The adjusted solution (1.0 kg) was placed in a 2.0 L autoclave (NAC Autoclave Co., Ltd.), and 20 g of Raney nickel (R239, product of Nikko Rika) was added. After adjusting a pH to 9.6 with 7.2% disodium phosphate solution and 21% sodium hydroxide solution, hydrogen gas was introduced for 2 hours at 130 °C and 100 kg/cm² to perform a hydrogenation reaction. After the reaction, the Raney nickel was removed, the reaction product was decolorized by activated charcoal, desalinated by the mixed ion-exchange resin column and then concentrated. The composition of the concentrated liquid thus obtained was 10.1 parts of mannitol, 31.1 parts of sorbitol, and 58.7 parts of hydrogenated indigestible dextrin. (It is well known to skilled in the art that equal amounts of mannitol and sorbitol can be produced by hydrogenating fructose.) The energy of the hydrogenated indigestible dextrin comprising hydrogenated isoglucose was 1.5 kcal/g. A relative sweetness of the hydrogenated indigestible dextrin to that of sugar whose sweetness is 100 was 30.

### Example 7

Candy was prepared using the hydrogenated indigestible dextrin comprising hydrogenated isoglucose obtained in Example 6. After adjusting the solution containing 200 g solids to a Brix concentration of 60%, the mixture was placed in a stainless steel vessel, electrically heated while gently being stirred until it was heated to a temperature of 160 °C and then boiled down. The mixture was then allowed to cool to about 80 °C and poured into a mold to obtain a candy. The candy thus obtained was released well from the mold, was clear in appearance with a surface free of irregularities with an appropriate amount of firmness while being chewed. It had low moisture absorbency as well as superior storage stability and heat stability.

### Brief Description of the Drawings

(Figure 1) This is an HPLC chart of the analysis of the mixed solution of glucose and indigestible dextrin obtained in the enzyme digestion of pyrodextrin in Example 1.
(Figure 2) This is an HPLC chart of the analysis of the mixed solution of glucose and indigestible dextrin obtained in the enzyme digesting of pyrodextrin in Example 1 to which fructose was added so that a ratio of fructose to glucose was 30.0 parts to 41.4 parts.
(Figure 3) This is an HPLC chart of the analysis of the mixed solution of glucose and indigestible dextrin obtained in the enzyme digesting of pyrodextrin in Example 1 to which fructose was added so that a ratio of fructose to glucose was 50.6 parts to 41.4 parts.
(Figure 4) This is an HPLC chart of the analysis of the mixed solution of isoglucose and indigestible dextrin obtained in Example 2 by acting glucose isomerase on a mixed solution of glucose and indigestible dextrin.
(Figure 5) This is an HPLC chart of the analysis of isoglucose liquid obtained in a control test in Example 2.
(Figure 6) This is an HPLC chart of the analysis of a mixed solution of isoglucose and indigestible dextrin obtained by acting glucose isomerase at SV 1.2 on a mixed solution of glucose and indigestible dextrin in Example 3.
(Figure 7) This is an HPLC chart of the analysis of isoglucose liquid obtained in the control test in Example 3.
(Figure 8) This is an HPLC chart of the analysis of a mixed solution of isoglucose and indigestible dextrin obtained by acting glucose isomerase at SV 1.75 on a mixed solution of glucose and indigestible dextrin in Example 3.

## Claims

1. A method of producing a indigestible dextrin comprising isoglucose, which comprises the steps of acting glucoamylase on an aqueous solution of pyrodextrin and then acting glucose isomerase and/or adding fructose.

2. The method of claim 1, wherein α-amylase is acted before the glucoamylase is acted.

## Patentansprüche

1. Ein Verfahren zur Herstellung eines unverdaulichen Dextrins umfassend Isoglucose, das die Schritte des Wirken lassens von Glucoamylase auf eine wässrige Lösung von Pyrodextrin und dann des Wirken lassens von Glucoseisomerase und/oder Zugebens von Fructose umfasst.

2. Das Verfahren nach Anspruch 1, wobei man α-Amylase wirken lässt bevor man die Glucoamylase wirken lässt.

## Revendications

1. Procécé de production d'une dextrine indigeste comprenant de l'isoglucose, qui comprend les étapes de faire agir une glucoamylase sur une solution aqueuse de pyrodextrine puis de faire agir une glucose isomérase et/ou d'ajouter du fructose.

2. Procédé selon la revendication 1 où l'α-amylase est mise à agir avant que la glucoamylase soit mise à agir.
